(19) **Europäisches Patentamt**

**European Patent Office**

**Office européen des brevets**

(11) Veröffentlichungsnummer : **0 040 397**
**B1**

# (12) EUROPÄISCHE PATENTSCHRIFT

(45) Veröffentlichungstag der Patentschrift :
25.07.84

(21) Anmeldenummer : 81103686.2

(22) Anmeldetag : 13.05.81

(51) Int. Cl.³ : **B 01 J 19/24, C 07 G 7/00**

---

(54) **Verfahren zur Herstellung von Blutplasma-Fraktionen.**

---

(30) Priorität : 16.05.80 DE 3018669

(43) Veröffentlichungstag der Anmeldung :
25.11.81 Patentblatt 81/47

(45) Bekanntmachung des Hinweises auf die Patenterteilung : 25.07.84 Patentblatt 84/30

(84) Benannte Vertragsstaaten :
**AT BE CH DE FR GB IT LI NL SE**

(56) Entgegenhaltungen :
**DE-A- 2 444 524**
**DE-A- 2 516 284**
**Chemical Abstracts Band 89, Nr. 17, 23. Oktober 1978 Columbus, Ohio, USA N.E. LEE et al. "Automated system for fractionation of blood samples" Seite 262, Zusammenfassung Nr. 142715y**
**Chemical Abstracts Band 93, Nr. 15, 13. Oktober 1980 Columbus, Ohio, USA UNITED STATES DEPT. OF COMMERCE "Centrifuge apparatus for blood cells" Seite 346, Zusammenfassung Nr. 145815z**

(73) Patentinhaber : **BEHRINGWERKE AKTIENGESELL-SCHAFT**
**Postfach 1140**
**D-3550 Marburg/Lahn (DE)**

(72) Erfinder : **Falke, Jürgen, Dr.**
**Oberer Eichweg 22**
**D-3550 Marburg (DE)**
Erfinder : **Geiger, Helmut, Dr.**
**Albert-Demnitz-Weg 5**
**D-3550 Marburg (DE)**
Erfinder : **Grünbein, Wolfgang, Dr.**
**Alt-Oberliederbach 35**
**D-6237 Liederbach (DE)**
Erfinder : **Kandel, Heinz-Georg**
**Gartenweg 6**
**D-3552 Wetter (DE)**

(74) Vertreter : **Meyer-Dulheuer, Karl-Hermann, Dr. et al**
**HOECHST Aktiengesellschaft Zentrale Patentabteilung Postfach 80 03 20**
**D-6230 Frankfurt/Main 80 (DE)**

EP 0 040 397 B1

## Beschreibung

Die vorliegende Erfindung betrifft ein Verfahren zur Herstellung von Plasmafraktionen durch Fällung.

Für manche Anwendungen in der Human- und Veterinärmedizin werden Albumine und Globuline verwendet. Diese Proteine werden durch fraktionierte Fällung aus menschlichem oder tierischem Plasma erhalten. Bisher wurde die Fällung in der Weise durchgeführt, daß man ansatzweise das Plasma vorlegte und durch langsames Eindosieren des Fällungsmittels die einzelnen Fraktionen gewann. Die Nachteile dieses diskontinuierlichen Verfahrens bestehen darin, daß es wegen der langen Verweilzeiten sehr zeitaufwendig ist und dennoch schwer abtrennbare Niederschläge ergibt.

Es bestand daher die Aufgabe, ein Fraktionierungsverfahren zu finden, welches in angemessener Zeit gut abtrennbare Fällungen liefert.

Es wurde gefunden, daß diese Aufgabe gelöst werden kann, wenn das zu fraktionierende Plasma, das Fällmittel und der Puffer im Kreislauf geführt wird.

Gegenstand der Erfindung ist somit ein Verfahren zur Herstellung von Plasma-Fraktionen durch Mischen von Plasma mit einem Fällungsmittel, bei konstanter Temperatur und in Gegenwart eines Puffers, und Abtrennen des ausgefällten Plasmaproteins, dadurch gekennzeichnet, daß das Gemisch aus Plasma, Fällmittel, Puffer und ausgefälltem Protein in einem Schlaufenreaktor, welcher aus einem mit einer Umlaufpumpe verbundenen Rohrkreislauf besteht, geführt, laufend ein Teil dieser Mischung entnommen und gleichzeitig eine entsprechende Menge an zu fraktionierendem Medium, Fällmittel und Puffer wieder zugeführt wird.

Ein besonderes Merkmal des Verfahrens ist, daß das Gemisch aus Plasma, Fällmittel, Puffer und ausgefälltem Protein in einem Schlaufenreaktor, welcher aus einem Rührbehälter besteht, dessen Ablauf mit dem Eingang einer Umlaufpumpe und dessen Zulauf mit dem Ausgang dieser Umlaufpumpe verbunden ist, im Kreislauf geführt wird, das Plasma, das Fällmittel und der Puffer getrennt in einer Zone hoher Verwirbelung dem System zugeführt werden, und durch eine zwischen Rührbehälterablauf um Pumpeneingang angeordnete Abzweigleitung laufend einer der zudosierten Menge an Plasma, Fällmittel und Puffer entsprechenden Menge Reaktionsgemisch dem System entnommen wird.

Das erfindungsgemäße Verfahren wird in einer Art « Schlaufenreaktor » durchgeführt. Dieser Schlaufenreaktor besteht aus einem Rührbehälter und einer Kreislaufleitung, in welche eine Umlaufpumpe eingeschaltet ist.

Ein weiteres Kennzeichen des Verfahrens ist, daß das dem Schlaufenreaktor entnommene Reaktionsgemisch vor der Abtrennung des ausgefällten Proteins eine Verweilzeitstrecke durchfließt.

Der Rührbehälter ist von der üblichen Art und mit Einrichtungen zum Messen und Regeln der Temperatur und des pH-Wertes des Fällmediums und zum Heizen bzw. Kühlen und Umwälzen des Inhaltes ausgerüstet. Das Heizen bzw. Kühlen kann durch einen Doppelmantel, äußere oder innere Rohrschlangen udgl. erfolgen. Der Behälterinhalt wird mittels eines durch einen Motor angetrieben Rührers, durch Einleiten eines Inertgases oder durch den Impuls des zulaufenden Fällmediums umgewälzt. Zulauf und Ablauf für das im Kreislauf zu führende Medium können beliebig angeordnet sein, vorzugsweise wird jedoch der Ablauf am Behälterboden angebracht und der Zulauf endet von oben kommend unter dem Flüssigkeitsspiegel. Der Behälterablauf ist mit dem Eingang einer Umlaufpumpe, der Ausgang dieser Pumpe mit dem Behälterzulauf verbunden. Zwischen Behälterablauf und Pumpeneingang ist eine Abzweigleitung angeordnet, welche mit einem Ventil abgesperrt werden kann und zu einer Abscheideeinrichtung, z. B. einem Filter oder einer Zentrifuge führt. Zu einer Zone besonders hoher Verwirbelung, z. B. am Pumpeneingang, führen auch noch die Zuleitungen von den Dosierpumpen für das zu fraktionierende Medium, für das Fällmittel und für den Puffer. Als Umlaufpumpe wird eine Pumpe mit großer Förderleistung, vorzugsweise eine stopfbuchslose Kreiselpumpe verwendet, als Dosierpumpen sind Schlauch-, Zahnrad-, Kolben- oder Membranpumpen geeignet.

Je nach Größe der Apparatur werden auch die Rohrleitungen und Pumpen durch Doppelmantel, Heiz- und Kühlschlangen oder dgl. temperiert oder der gesamte Schlaufenreaktor mit den Dosierpumpen wird in einem temperierten Raum untergebracht.

Als Material für die Apparatur eignen sich Glas, Emaille, korrosionsfester Kunststoff oder Metall, z. B. Edelstahl. Die Materialauswahl richtet sich in erster Linie nach der Größe der Apparatur. Während kleine Apparaturen ganz aus Glas und/oder Kunststoff angefertigt werden können, sind für große Apparaturen Metall, Glas oder Emaille vorzuziehen.

Die Größe des Rührbehälters richtet sich nach den umzusetzenden Mengen, sie reicht von 0,5 l bis 100 l, vorzugsweise von 0,5 l bis 10 l. Die Maße der anderen Apparateteile sind der Größe des Rührbehälters jeweils angepaßt.

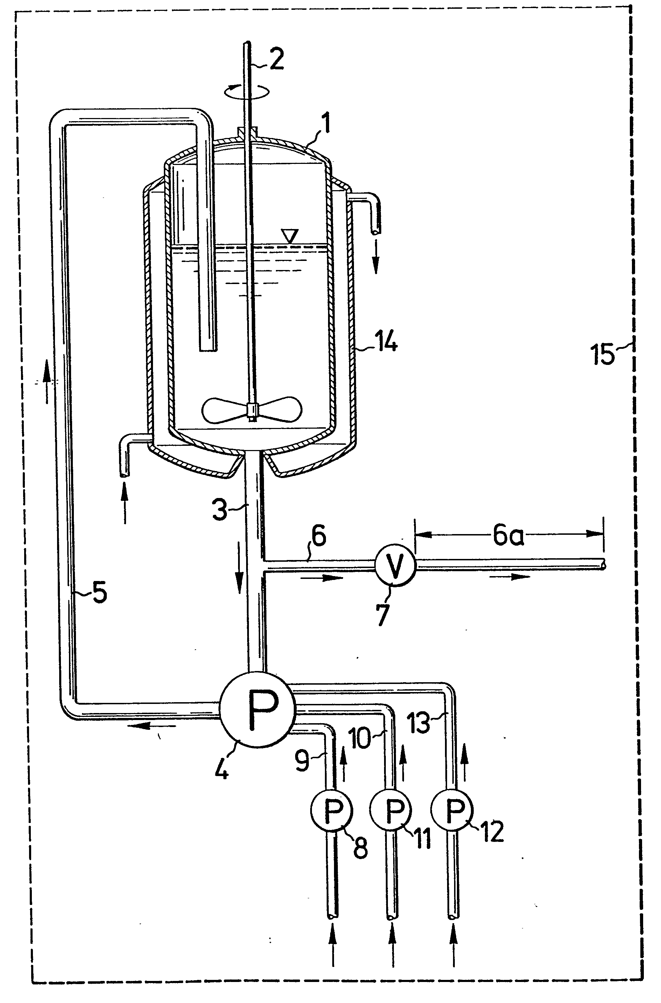

Aus der Figur ist der bevorzugte Aufbau einer erfindungsgemäß zu verwendenden Apparatur ersichtlich. Der Rührbehälter (1) mit Rührwerk (2) ist über die Rohrleitung (3) mit der Umlaufpumpe (4) verbunden. Von der Pumpe (4) führt die Rohrleitung (5) wieder in den Rührbehälter (1) zurück. Von der Leitung (3) zweigt die Rohrleitung (6) ab und führt über die Verweilzeitstrecke (6a) zur nicht gezeigten Abscheideeinrichtung. Diese Leitung (6) ist durch das Ventil (7) absperrbar. Mit dem Eingang der Pumpe (4) sind außerdem die Dosierpumpen (8), (11) und (12) über die Rohrleitungen (9), (10) und (13) verbunden. Der Rührbehälter wird mittels eines Doppelmantels (14) temperiert. Die ganze Apparatur kann sich auch in einem temperierten Raum befinden, der durch die gestrichelte Linie (15) angedeutet ist.

Das erfindungsgemäße Verfahren wird vorzugsweise für die Fraktionierung von menschlichem oder tierischem Plasma bzw. Plasmafraktionen angewendet, jedoch ist es auch für die Fraktionierung anderer Eiweißstoffe anwendbar.

Das Fällmittel ist dem zu fällenden Material anzupassen, für menschliches oder tierisches Plasma wird vorzugsweise (NH$_4$)$_2$SO$_4$-Lösung, Alkohol, wie z. B. Ethanol, oder ein Polyätheralkohol, wie z. B. Polyethylenglykol, verwendet. Auch die Menge des Fällmittels richtet sich nach der Art und der Menge der zu fällenden Eiweißfraktionen und ist durch einen Vorversuch zu ermitteln.

Zum Konstanthalten des für die Fällung optimalen pH-Wertes dient eine Säure oder Lauge, bzw. ein handelsüblicher Puffer.

Das Gemisch aus zu fraktionierendem Medium, Fällmittel, Puffer und ausgefälltem Protein wird mittels der Umlaufpumpe dem Rührbehälter entzogen und an anderer Stelle wieder zugeführt. Währenddessen wird am Abzweig laufend ein Teil dieser Mischung entnommen und in der Abscheideeinrichtung vom gefällten Protein abgetrennt. Gleichzeitig wird die entsprechende Gesamtmenge an zu fraktionierendem Medium, Fällmittel und Puffer dem System durch die Dosierpumpen wieder zugeführt, so daß im System eine weitgehend ausgeglichene Stoffbilanz herrscht. Das Zudosieren muß dabei nicht kontinuierlich erfolgen, sondern kann intermittierend durchgeführt werden. Wichtig ist, daß die Konzentrationsschwankungen im gesamten Schlaufenreaktor innerhalb der tolerierbaren Grenzen für die abzuscheidende Protein-Fraktion liegen.

Durch das erfindungsgemäße Verfahren werden Protein-Flocken gewonnen, die sich leicht abtrennen lassen. Bei Benutzung einer Zentrifuge kann ein kontinuierlich entleerbares Gerät verwendet werden, da die erforderlichen Zentrifugalkräfte zur Abscheidung der Protein-Flocken sehr niedrig sind. Durch die kurze Verweilzeit im Schlaufenreaktor kann der Durchsatz durch die Apparatur auf das Mehrfache des Durchsatzes bei dem bekannten Fällungsverfahren gesteigert werden. Weiterhin hat sich gezeigt, daß die bei längerer Betriebsdauer zu befürchtende erhöhte Verkeimungsgefahr nicht besteht.

Beispiel 1

Der Rührbehälter (1) gemäß der Figur hat einen Inhalt von 1,5 l, ist doppelwandig besteht aus Glas und wird auf − 2 °C temperiert. Die Leitungen (3) und (5) bestehen aus isolierten Glas-Doppelrohren. Die stopfbuchlose Kreiselpumpe (4) wälzt den Inhalt des Rührbehälters (1) und der Leitungen (3) und (5) mit einer Förderleistung von 200 l/h um. Die Schlauchpumpen (8), (11) und (12) führen dem System 800 ml/h eines Humanplasmas, 93 ml/h Ethanol und soviel eines handelsüblichen Puffers (pH 4,8) zu, daß der pH-Wert im System 7,4 beträgt. An dem Ventil (7) werden kontinuierlich 900 ml/h Reaktionsflüssigkeit abgezogen, aus der man durch Zentrifugieren ca 10 g/h einer angereicherten Fibrinogenfraktion abtrennt.

Beispiel 2

In eine gleiche Apparatur wie in Beispiel 1, jedoch mit einem doppelwandigen Glasgefäß von 3,0 l Inhalt als Rührbehälter (1) werden bei einer Temperatur von − 6 °C und einem pH-Wert von 7,6 800 ml/h Human-Plasma, 280 ml/h Ethanol sowie Puffer-Lösung zugeführt und 1 000 ml/h Reaktionsflüssigkeit abgezogen. Man gewinnt ca. 50 g/h einer angereicherten Immunglobulinfraktion.

**Ansprüche**

1. Verfahren zur Herstellung von Plasma-Fraktionen durch Mischen von Plasma mit einem Fällmittel, bei konstanter Temperatur und in Gegenwart eines Puffers, und Abtrennen des ausgefällten Plasmaproteins, dadurch gekennzeichnet, daß das Gemisch aus Plasma, Fällmittel, Puffer und ausgefälltem Protein in einem Schlaufenreaktor, welcher aus einem mit einer Umlaufpumpe verbundenen Rohrkreislauf besteht, geführt, laufend ein Teil dieser Mischung entnommen und gleichzeitig eine entsprechende Menge an zu fraktionierendem Medium, Fällmittel und Puffer wieder zugeführt wird.

2. Verfahren nach Anspruch 1, dadurch gekennzeichnet, daß das Gemisch aus Plasma, Fällmittel, Puffer und ausgefälltem Protein in einem Schlaufenreaktor, welcher aus einem Rührbehälter besteht, dessen Ablauf mit dem Eingang einer Umlaufpumpe und dessen Zulauf mit dem Ausgang dieser Umlaufpumpe verbunden ist, im Kreislauf geführt wird, das Plasma, das Fällmittel und der Puffer getrennt in einer Zone hoher Verwirbelung dem System zugeführt werden, und durch eine zwischen Rührbehälterablauf und Pumpeneingang angeordnete Abzweigleitung laufend eine der zudosierten Menge an Plasma, Fällmittel und Puffer entsprechende Menge Reaktionsgemisch dem System entnommen wird.

3. Verfahren nach Anspruch 1, dadurch gekennzeichnet, daß das dem Schlaufenreaktor entnommene Reaktionsgemisch vor der Abtrennung des ausgefällten Proteins eine Verweilzeitstrecke durchfließt.

**Claims**

1. A process for the preparation of plasma fractions by mixing plasma with a precipitant, at constant temperature and in the presence of a buffer, followed by separation of the precipitated plasma protein, which comprises circulating the mixture consisting of plasma, precipitant, buffer and precipitated protein in a loop reactor consisting of a closed tube circuit connected with a circulating pump, withdrawing continuously part of said mixture and reintroducing simultaneously a corresponding quantity of medium to be fractionated, of precipitant and of buffer.

2. The process as claimed in claim 1, which comprises circulating the mixture consisting of plasma, precipitant, buffer and precipitated protein in a loop reactor consisting of a stirred tank, the outlet of which is connected with the inlet of a circulating pump and the inlet of which is connected with the outlet of this circulating pump, separately conveying the plasma, the precipitant and the buffer to the system in a zone of a high turbulence and withdrawing continuously from the system a quantity of reaction mixture, corresponding to the feed quantity of plasma, precipitant and buffer, through a branch pipe located between the outlet pipe of the stirred tank and the inlet pipe of the pump.

3. The process as claimed in claim 1, wherein the reaction mixture withdrawn from the loop reactor flows through a dwelling zone prior to the separation of the precipitated protein.

## Revendications

1. Procédé de préparation de fractions de plasma par mélange de plasma avec un agent de précipitation, à température constante et en présence d'un tampon, et séparation de la protéine plasmatique précipitée, caractérisé en ce qu'on envoie le mélange de plasma, d'agent de précipitation, de tampon et de protéine précipitée dans un réacteur à boucle, qui se compose d'un circuit de tubes relié à une pompe de circulation, en ce qu'on prélève en continu une partie de ce mélange et renvoie simultanément une quantité correspondante de milieu à fractionner, d'agent de précipitation et de tampon.

2. Procédé suivant la revendication 1, caractérisé en ce qu'on envoie en circuit fermé le mélange de plasma, d'agent de précipitation, de tampon et de protéine précipitée dans un réacteur à boucle qui se compose d'un récipient à agitation, dont la sortie est reliée à l'entrée d'une pompe de circulation et dont l'entrée est reliée à la sortie de cette pompe de circulation, en ce qu'on envoie séparément dans le système le plasma, l'agent de précipitation et le tampon dans une zone à forte turbulence, et en ce qu'on prélève en continu dans le système, par une canalisation de dérivation disposée entre la sortie du récipient à agitation et l'entrée de la pompe, une quantité de mélange réactionnel correspondant à la quantité de plasma, d'agent de précipitation et de tampon introduite.

3. Procédé suivant la revendication 1, caractérisé en ce que le mélange réactionnel prélevé dans le réacteur à boucle traverse un parcours de temps de séjour avant la séparation de la protéine précipitée.